# EUROPEAN PATENT SPECIFICATION

(11) **EP 0 441 353 B1**
(45) Date of publication and mention of the grant of the patent: **10.01.2001**
(21) Application number: 91101620.2
(22) Date of filing: 06.02.1991
(51) Int. Cl.: C12N 15/54, C12N 9/10

(54) **Process for the production of gamma-glutamyl transpeptidase**
Verfahren zur Herstellung von gamma-Glutamylpeptidase
Procédé pour la production de transpeptidase gamma-glutamyle

(30) Priority: 07.02.1990 JP 2774090
(43) Date of publication of application: 14.08.1991
(73) Proprietor: KYOWA HAKKO KOGYO CO., LTD., Chiyoda-ku, Tokyo-to (JP)
(72) Inventor: Ryoichi, Katsumata, Tokyo (JP); Toru, Mizukami, New York 11743 (US); Shigenori, Ohta, Tokyo (JP); Moriyuki, Sato, Tokyo (JP); Kazuo, Yamaguchi, Kanagawa-ken (JP)
(74) Representative: VOSSIUS & PARTNER

(56) References cited:
- EP-A- 0 275 901
- EP-A- 0 321 849
- JOURNAL OF BACTERIOLOGY, vol. 171, no. 9, September 1989, pages 5169-5172; H. SUZUKI et al.: "DNA sequence of the Escherichia coli K-12 gamma glutamyl transpeptidase gene, ggt"
- PROC. NATL. ACAD. SCI. USA, vol. 85, December 1988, pages 8840-8844; E. RAJPERT-DE MEYTS et al.: "Cloning and nucleotide sequence of human gamma-glutamyl transpeptidase"
- SUZUKI ET AL.: "", BIOCHEM. BIOPHYS. RESEARCH COMMUNICATIONS, , 15-01-1988, vol. 150, no. 1, pages 33 to 38
- HARA ET AL.: "", J. GEN. APPL. MICROBIOL., , , vol. 27, no. , pages 299 to 305
- SUZUKI ET AL.: "", J. BACTERIOL., , , vol. 168, no. 3, pages 1325 to 1331

## Description

This invention relates to a process for the production of γ -glutamyl transpeptidase (EC 2.3.2.2), having the nucleotide sequence as defined in the sequence listing by SEQ ID:No. 1 and encoding a polypeptide having the γ -glutamyl transpeptidase activity of a bacterium of the genus Bacillus.

γ -Glutamyl transpeptidase is an enzyme which catalyzes (1) hydrolysis of glutathione to produce glutamic acid or (2) transfer of the γ -glutamyl group of glutathione to an amino acid or peptide, as follows:
(1) Hydrolysis: ${\text{γ-Glu-Cys-Gly + H}}_{\text{2}} \text{O → Glu + Cys-Gly}$
(2) Transfer: $\text{γ-Glu-Cys-Gly + A → γ-Glu-A + Cys-Gly}$
(wherein A is an amino acid or a peptide)

γ -Glutamyl transpeptidase is useful as a dough conditioner for increasing the specific volume, for improving the content, and for suppression of retrogradation of bread [Japanese Patent Application LOP Publn. No. 1985-2,135].

γ -Glutamyl transpeptidase is known to be widely present in various organisms from bacteria to higher animals [A. Meister and S. Tate: Ann. Rev. Biochem., 45, 559 (1976)]. The enzyme has been isolated and purified mainly from the organs of higher animals. The production and purification of γ -glutamyl transpeptidase from bacterial origin have been studied on Proteus mirabilis and Escherichia coli [H. Kumagai et al., J. Bacteriol., 160, 341 (1984) and H. Suzuki et al., J. Bacteriol., 168, 1325 (1986)]. The cloning of a γ-glutamyl transpeptidase gene derived from Escherichia coli has also been reported [H. Suzuki et al., Biochem. Biophys. Res. Commun., 150, 33 (1988)]. Its characterization by means of DNA sequencing has also been described [H. Suzuki et al., J. Bacteriol., 171, 5169 (1989)]. The use of the γ -glutamyl transpeptidase derived from such bacterial origins as mentioned above as an additive for food is undesirable from the standpoint of safety. It has been reported that Bacillus natto [Debabov. V. G., "The Molecular Biology of the Bacilli," 1, 331, Academic Press (1982)] and Bacillus licheniformis, known to possess very high level of safety, produce a γ -glutamyl transpeptidase [Hara and Ueda: "Fermentation and Industry, 43, 910 (1985) and Asada et al., Summary of Lectures at 1989 General Meeting of Japan Society for Bioscience, Biotechnology and Agrochemistry p.125]. It has also been reported that Bacillus subtilis, a species very closely related to Bacillus natto, produces an enzyme having a γ -glutamyl transferase activity [W. Williams and C. B. Throne, J. Biol. Chem., 210, 203 (1954)]. However, the yield of the enzyme from the genus Bacillus is poor and the genus Bacillus is unsuitable for the enzyme production.

The method of producing the enzyme from a cell culture of the genus Pseudomonas, the genus Proteus, the genus Arthrobacter or Bacillus subtilis has been disclosed [Japanese Patent Application LOP - Publn. No. 1988 - 181, 996]. However, the yield thereof is low and the bacteria except for Bacillus subtilis are not suitable for producing an enzyme intended for use in food from the standpoint of safety. Furthermore, the above mentioned microorganisms are unsuitable for industrial production since further purification is needed to remove contaminating proteins due to the destructive process of cells and cellular debris.

An object of the invention is to provide a bacterial strain capable of producing γ -glutamyl transpeptidase in high yield and without toxic contaminants. The enzyme is useful as a dough conditioner. Another object of the invention is to provide a method for the inexpensive and simple production of the enzyme using said bacterial strain.

In the recent progress of molecular biology, a genetically engineered bacterium is generally used for producing useful proteins and enzymes. The bacterium containing a gene encoding such a protein or enzyme is cultured to express the gene product. When genetic engineering is utilized for such a production, it is essential to select a suitable and safe host bacterium and a simple and efficient method of purifying the protein produced. The genus Bacillus is characterized by secreting a large amount of protein and Bacillus subtilis, a species closely related to Bacillus natto, is one of the safest, non-pathogenic species.

The inventors have screened a group of strains belonging to Bacillus subtilis to find a strain capable of producing and secreting γ -glutamyl transpeptidase activity and found Bacillus subtilis SJ138 as a γ-glutamyl transpeptidase-producing strain. Since the SJ138 strain has a poor γ -glutamyl transpeptidase-producing ability, we have tried to improve the productivity thereof. We have cloned the gene of γ -glutamyl transpeptidase from the strain and self-cloned it into a plasmid vector of Bacillus subtilis, for example, plasmid pUB110 or a derivative thereof to construct a recombinant plasmid. A bacterium of the genus Bacillus such as of the γ -glutamyl transpeptidase-producing strain SJ138 or of the non-producing strain Bacillus subtilis Marburg BR151 [ATCC 33677, D. M. Williams et al., J. Bacteriol., 146, 1162 (1981)] can be transformed with the recombinant molecule. The transformant of a SJ138 host contains multiple copies of the gene and produces a higher yield of the enzyme than the original strain SJ138.

The invention provides a gene which codes for γ -glutamyl transpeptidase and has the nucleotide sequence as defined in the Sequence Listing by SEQ ID : No. 1.

The invention further provides a recombinant DNA molecule comprisedof a vector DNA and a DNA fragment containing the gene coding for γ -glutamyl transpeptidase and having the nucleotide sequence as defined in the sequence listing by SEQ ID:No. 1 and encoding a polypeptide having the γ -glutamyl transpeptidase activity of a bacterium of the genus Bacillus.

The invention further provides a microorganism carrying a recombinant DNA molecule comprised of a vector DNA and a DNA fragment containing the gene coding for γ -glutamyl transpeptidase and having the nucleotide sequence as defined in the sequence listing by SEQ ID:No. 1 and encoding a polypeptide having the γ -glutamyl transpeptidase activity of a bacterium of the genus Bacillus. Preferably the microorganism is of the genus Bacillus.

The present invention further provides a process for producing γ -glutamyl transpeptidase, which comprises culturing in a suitable culture medium a microorganism carrying a recombinant DNA comprised of a vector DNA and a DNA fragment which contains a gene coding for γ -glutamyl transpeptidase and having the nucleotide sequence as defined in the sequence listing by SEQ ID:No. 1 and encoding a polypeptide having the γ -glutamyl transpeptidase activity of a bacterium of the genus Bacillus, accumulating γ -glutamyl transpeptidase in the culture, and recovering γ -glutamyl transpeptidase therefrom.

The figures show:

Fig. 1: Cloning strategy of a γ -glutamyl transpeptidase gene into a cloning vector of Bacillus subtilis and the construction of plasmid pG31.

Fig. 2: Method of constructing plasmids pEX653 and pUG55.

Fig. 3: Restriction map of the cloned γ -glutamyl transpeptidase gene in pG31.

In the Figures, E is EcoRI, X is XbaI, B is BamHI, S is SphI, B/Sa is a BamHI/Sau3A ligation site, Sc is SacI, K is Kpnl, Sm is Smal, Sl is SalI, P is PstI, H is HindIII, N/Sm is a NruI/SmaI ligation site, C is ClaI, He is HincII, Km is Kanamycin-resistant marker, Cm is Chloramphenicol-resistant marker, and Ap is Ampicillin resistant marker.

The invention is described in detail herein-after.

Both a DNA fragment containing the gene encoding γ -glutamyl transpeptidase and having the nucleotide sequence as defined in the sequence listing by SEQ ID:No. 1 and encoding a polypeptide having the γ -glutamyl transpeptidase activity of a bacterium of the genus Bacillus, and a plasmid vector are derived from the genus Bacillus. The source of the gene is chromosomal DNA and the gene is inserted into the vector, which is duplicated in the genus Bacillus, using conventional recombinant DNA technology.

Concrete examples of bacteria which can produce and secrete γ -glutamyl transpeptidase include Bacillus subtilis SJ138 (FERM BP-2694), Bacillus subtilis ATCC9372, and Bacillus brevis ATCC10027. These strains are selected from various Bacillus strains by measuring γ - glutamyl transpeptidase activity in the supernatant of their culture. The γ -glutamyl transpeptidase activity can be determined using γ -glutamyl-p-nitroanilide as a substrate and measuring the amount of p-nitroaniline produced by the enzyme reaction using a colorimeter at 410nm.

The cloning of the γ -glutamyl transpeptidase gene derived from Bacillus subtilis SJ138 is described below Bacillus subtilis SJ138 was deposited with the Fermentation Research Institute, Agency of Industrial Science and Technology on December 21, 1989 under the Budapest Treaty and assigned the accession number FERM BP-2694.

The steps to clone the γ -glutamyl transpeptidase gene of Bacillus subtilis SJ138 are as follows:
(a) Isolation and purification of the γ -glutamyl transpeptidase,
(b) Determination of the amino acid sequence of the γ -glutamyl transpeptidase,
(c) Synthesis of a DNA probe corresponding to the amino acid sequence,
(d) Detection of the γ -glutamyl transpeptidase gene on the chromosomal DNA of SJ138 using the probe, and
(e) Cloning of the gene into a suitable vector.

### (1) Isolation and purification of the γ -glutamyl transpeptidase

The γ -glutamyl transpeptidase can be purified by the standard method. γ -Glutamyl transpeptidase may be purified from the supernatant of a culture by fractional precipitation using neutral salts, organic solvents, etc., or by ion exchange chromatography. The fractions eluted from the column are tested for the γ -glutamyl transpeptidase activity and the active fractions are collected. For example, γ -glutamyl transpeptidase may be purified from the supernatant of a culture by ethanol precipitation, lyophilization, DEAE-cellulose column chromatography and hydroxyapatite column chromatography. The determination of the molecular weight of the purified γ -glutamyl transpeptidase from SJ138 by gel filtration and SDS-polyacrylamide gel electrophoresis suggests that the enzyme is a dimer consisting of about 41 KDa (kilo dalton) and 23 KDa subunits (1:1).

### (2) Determination of the amino acid sequence of γ - glutamyl transpeptidase

The purified γ -glutamyl transpeptidase is separated into two subunits using reverse high pressure liquid chromatography. Each of the subunits is fragmented using a protease, for example trypsin, and the fragments are fractionated by high pressure liquid chromatography. The amino acid sequences of the peptide fragments are analyzed by the standard method using an amino acid sequencer.

### (3) Synthesis of an oligonucleotide probe corresponding to the amino acid sequence of γ -glutamyl transpeptidase

The oligonucleotides about 15 -18 bases long and corresponding to a sequence of 5 - 6 amino acids of the enzyme are synthesized to be used as a probe for colony hybridization. Since 1 - 6 codons correspond to a single amino acid, several oligonucleotides are prepared for the probe. It is desirable to select a region of the amino acid sequence where only a few oligonucleotides correspond to the amino acid sequence. One of the preferable probes corresponding to the sequence of the amino acids 5 - 10 of the partial sequence, as defined in the Sequence Listing by SEQ ID : No. 3 , of the 41 KDa subunit, is a mixture of 32 types of 17 bp oligonucleotides as defined in the Sequence Listing by SEQ ID No. 4. (where N is A, T , G or C, Y is T or C, and R is A or G) (designated as probe L). Another preferable probe includes a mixture of 64 types of 17 bp oligonucleotides as defined in the Sequence Listing by SEQ ID No. 7 designated as probes complementary to the oligonucleotide as defined in the Sequence Listing by SEQ ID No. 6 encoding the amino acids 2 - 7 of the partial sequence, as defined in the Sequence Listing by SEQ ID 5, of the 23 KDa subunit.

These oligonucleotides may be synthesized using a commercially available DNA synthesizer or the known solid phase phosphotriester method [H. Ito et al., Nucleic Acids Res., 10, 1755 (1982)]. The resultant oligonucleotides may be purified by high pressure liquid chromatography.

### (4) Southern hybridization of the chromonosamal γ -glutamyl transpeptidase gene of SJ138 using the synthesized DNA probe

Southern hybridization [E. M. Southern, J. Mol. Biol., 98 503 (1975)] is carried out to detect the chromosomal γ -glutamyl transpeptidase gene of SJ138 using the synthetic oligonucleotide probe described above. The sequences encoding the two subunits in the SJ138 chromosome may hybridize with the probes. The chromosomal DNA of SJ138 is digested with an appropriate restriction enzyme and fractionated by agarose gel electrophoresis. The DNA fragments are blotted onto a nitrocellulose filter, fixed, and then hybridized with the labelled probe.

Chromosomal DNA of SJ138 may be prepared from a culture according to the method of H. Saito and K. Miura [Biochim. Biophys. Acta 72, 619 (1963)]. Digestion of the chromosomal DNA with a restriction enzyme and agarose gel electrophoresis may be carried out according to standard methods [T. Maniatis et al., Molecular Cloning, Cold Spring Harbor Laboratory (1982)].

It has been found that both the probe L derived from the 41 KDa subunit and the probe S derived from the 23 KDa subunit hybridized with a 2.8 Kb Hind III fragment and a 2.9 Kb EcoRI fragment close to each other. These results suggest that the sequences encoding the two subunits are closely locatedin the SJ138 chromosome.

### (5) Cloning of the γ -glutamyl transpeptidase gene using a Bacillus subtilis host-vector system.

In a "shot gun" approach, the chromosomal DNA containing the γ -glutamyl transpeptidase gene and a plasmid vector derived from the genus Bacillus are first digested with an appropriate restriction enzyme. The resultant fragments are ligated and then the construct may be used to transform competent cells of Bacillus subtilis Marburg or protoplasts of Bacillus subtilis [J. Spizizen et al., Ann. Rev. Microbiol., 20, 371 (1966); T. Akamatsu and J. Sekiguchi, Agric. Biol. Chem., 46, 1617 (1982)]. The transformants are screened for γ-glutamyl transpeptidase activity. A nitrocellulose filter is placed on agar and the transformants are plated on the filter. When colonies are formed, colonies are transferred to a filter soaked with γ -glutamyl-p-nitroanilide, the substrate of γ -glutamyl transpeptidase. The enzyme-substrate reaction produces p-nitroaniline, a yellow substance. The transformants that show a yellow color around the colony are selected.

It had been known that chromosomal DNA transforms competent cell of Bacillus subtilis Marburg with higher efficiency than plasmid DNA [T. J. Gryczan et al., J. Bacteriol., 134, 138 (1978); S. Contente and D. Dubnau, Proc. Natl. Acad. Sci. USA 75, 1428 (1978)]. The increased number of transformants may be obtained according to the method described by S. Contente and D. Dubnau [Plasmid 2, 555 (1979)], in which competent cells of Bacillus subtilis Marburg are engineered to harbor a plasmid containing a partially homologous sequence to a plasmid to be used for transformation in order to induce homologous recombination. A transformant having γ-glutamyl transpeptidase activity is cultured and the recombinant plasmid DNA is purified by the method described by T. J. Gryczan et al. [J. Bacteriol., 134, 318 (1978)]. Using the probes L and S, Southern blot analysis of the plasmid DNA may indicate whether both sequences encoding two subunits are present in the plasmid. The source of the cloned sequence of the plasmid may be further examined by the nick translation method [P. W. J. Rigby et al.,J. Mol. Biol., 113, 237 (1977)] and Southern hybridization. Namely, the cloned sequence is radiolabelled and used for hybridization with chromosomal DNA of SJ138.

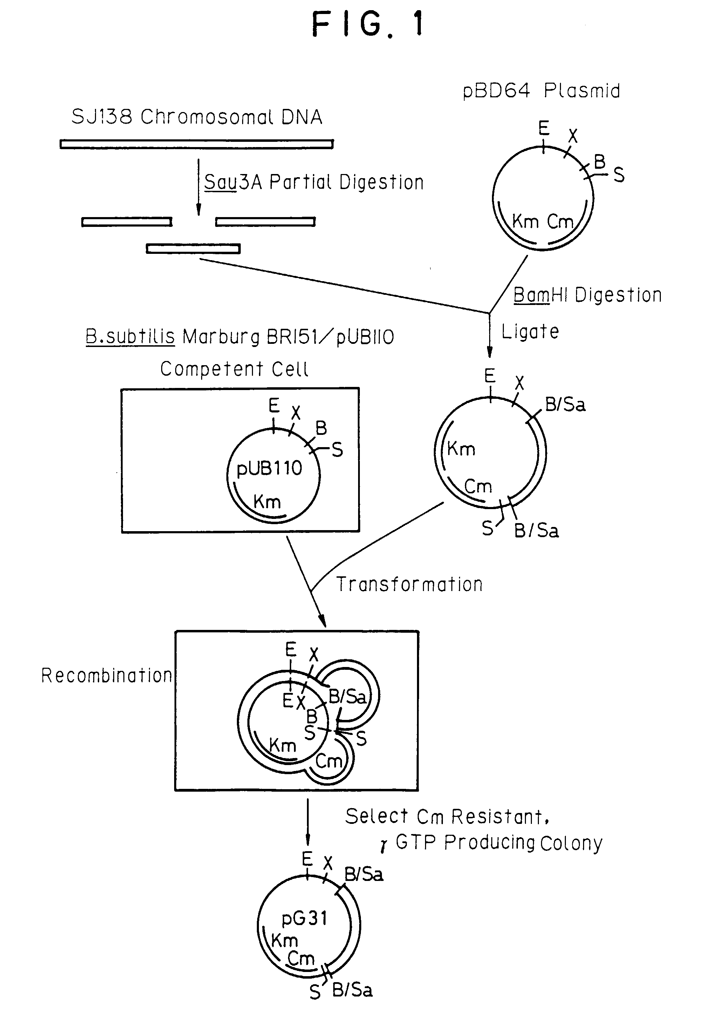

A Bam HI fragment of pBD64 [D. Dubnau et al., J. Bacteriol., 141, 206 (1980)] is ligated to fragments from a Sau 3A partial digest of SJ138 chromosomal DNA. The resulting construct may be used to transform Bacillus subtilis Marburg harboring pUB110 [T. Gryczan et al., J. Bacteriol., 134, 318 (1978)] containing a partially homologous sequence to that of p8D64. Recombinant plasmid pG31 containing the γ -glutamyl transpeptidase gene from the SJ138 chromosome is obtained as described above(See Fig 1).

The nucleotide sequence of the γ -glutamyl transpeptidase gene in pG31 is confirmed by DNA sequence analysis. A fragment containing the γ -glutamyl transpeptidase gene may be cleaved off from pG31, fragmented, and subcloned for sequencing. Alternatively, a single-stranded DNA may be synthesized from the subcloned fragment and sequenced. The sequencing may be carried out using the dideoxy chain termination method [J. Messing, Methods in Enzymology, 101, 20, Academic Press (1983)].

Vieira-Messing plasmids, namely E. coli plasmids pUC118 and pUC119 [J. Vieira and J. Messing, Methods in Enzymology, 153, 3 (1987)] may be used as vectors for the subcloning of the γ -glutamyl transpeptidase fragment. The size of the subcloned fragment may be reduced by exonuclease III digestion [S. Henikoff, Gene, 28, 351 (1984)]. The cloning vectors pUC118 and pUC119 contain the intergenic region (IG) of M13 phage [D. Mead and B. Kemper in "Vectors : A Survey of Molecular Cloning Vectors and Their Uses. "Butterworth, Massachusetts, (1986)]. Therefore, if the transformant E. coli MV1184 is infected with helper phage M13 K07, the IG may mediate the following events: (1) replication of double stranded DNA, (2) synthesis of the ⊕ single-stranded DNA using the ⊖ strand as a template, (3) packing of the ⊕ single-stranded DNA into M13 phage coat protein and budding out of the phage from the bacterial cell. The single-stranded DNA is prepared from the supernatant of the culture of MV1184 thus infected for sequencing using the dideoxy chain termination procedure [J. Vieira and J. Messing, Methods in Enzymology, 153, 3, Academic Press (1987)].

A single-stranded DNA may be also prepared by digesting the γ -glutamyl transpeptidase DNA fragment with restriction enzymes,ligating the resultant DNA fragment with a double-stranded M13mp18 or M13mp19 DNA [C. Yanisch-Perron et al., Gene, 33, 103 (1985)], infecting a suitable E. coli host with the recombinant phage, picking the recombinant plaques and growing them.

The open reading frame (ORF) encoding γ -glutamyl transpeptidase is identified by aligning the confirmed amino acid sequence and the corresponding nucleotide sequence.

The obtained ORF encoding γ -glutamyl transpeptidase DNA is as defined in the Sequence Listing by SEQ ID No. 1.

A single ORF has been found in the above DNA fragment according to the nucleotide sequence analysis. In the ORF, a signal sequence is in the upstream region (Yamane: Nippon Nogeikagakukai-shi, 61, 64, 1987) followed by a large subunit and a small subunit. Double digestion of the cloned DNA fragment with SphI and NruI generates a fragment of about 2.4 Kb, which may be used as an insert to construct a subclone free from foreign nucleotide sequences.

The self-clone free from foreign nucleotide sequences is constructed as described below.

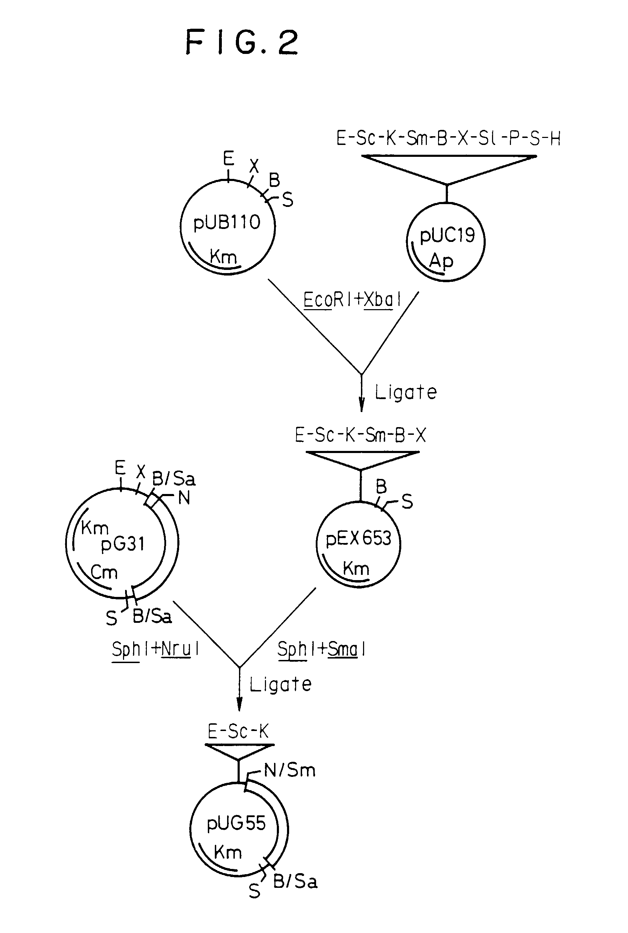

The vector DNA of the recombinant plasmid pG31 is derived from non-self type plasmid pBD64. The γ -glutamyl transpeptidase DNA fragment may be subcloned into a self-type plasmid of Bacillus subtilis origin such as pUB110 and the derivatives thereof. In the examples described below, pEX653, a derivative of pUB110 is used as a self-type plasmid. The vector pEX653 comprises an about 4.1 Kb EcoRI-Xba fragment of pUB110 ligated with a chemically synthesized linker copied from E. coli plasmid pUC19. The sequence of the linker is as defined in the Sequence Listing by SEQ ID No. 8. Multi copy plasmid pUG55, which encodes γ -glutamyl transpeptidase and whose host is a Bacillus subtilis Marburg BR151 strain, is a recombinant DNA molecule constructed by ligating an about 2.4 Kb SphI-NruI DNA fragment of pG31 with the SphI-SmaI fragment of pEX653 (See Fig 2). pUG55 may be introduced into SJ138 by the protoplast transformation method [T. Akamatsu and J. Sekiguchi, Agric. Biol. Chem. 46, 1617 (1982)].

The suitable strain carrying a recombinant plasmid containing a γ -glutamyl transpeptidase gene derived from the genus Bacillus is Bacillus subtilis SJ139 harboring pUG55 containing a γ -glutamyl transpeptidase gene derived from Bacillus subtilis SJ138. The strain Bacillus subtilis SJ139 was deposited with the Fermentation Research Institute, Agency of Industrial Science and Technology under the Budapest Treaty on December 21, 1989 and assigned the accession number FERM BP-2695.

γ -Glutamyl transpeptidase can be produced by culturing Bacillus subtilis carrying a recombinant plasmid containing a γ -glutamyl transpeptidase gene. Culturing of the microorganism may be carried out according to standard methods known in the art. Namely, the microorganism is cultured in a conventional medium at a suitable pH which contains carbon and nitrogen sources, minerals, amino acids, vitamines and the like, aerobically under temperature-and pH-control.

The carbon source includes carbohydrates such as glucose, fructose, sucrose, molasses, blackstrap molasses, starch hydrolysate, etc., alcohols such as ethanol, glycerol, sorbitol, etc., organic acid such as pyruvic acid, lactic acid, acetic acid, etc., and amino acids such as glycine, alanine, glutamic acid, aspartic acid, etc. Any materials that the microorganism can utilize may be used. The concentration of the carbon source in a culture medium is preferably 5 - 30% by weight.

The nitrogen source includes various inorganic and organic ammonium salts such as ammonium carbonate, ammonium acetate, ammonium phosphate, etc., nitrogen-containing organic materials such as urea, peptone, NZ amine, meat extract, yeast extract, corn steep liquor, casein hydrolysate, fish meal, digested fish meal, etc, and various amino acids such as glycine, glutamic acid etc. The concentration of the nitrogen source in a culture medium is typically in the range of 0.1 to 10% by weight.

Examples of minerals include potassium dihydrogen phosphate, dipotassium hydrogen phosphate, magnesium sulfate, magnesium phosphate, sodium chloride, ferrous sulfate, manganese sulfate, zinc sulfate, calcium carbonate, and the like. When specific materials such as amino acids, nucleic acids, vitamines, etc., are required for the growth of the microorganism an appropriate amount of these materials may be added to a culture medium.

The culture is grown aerobically with shaking or with aeration and agitation. The growth temperature preferably ranges between 20 - 40 °C.The incubation period may be 10 - 72 hours. It is desirable to keep the pH of the medium around neutral using ammonia, urea, or sodium hydroxide.

After the growth, γ -glutamyl-transpeptidase may be recovered from the culture medium using standard methods. The culture is centrifuged, and an organic solvent such as ethanol or aceton or an inorganic salt such as ammonium sulfate is added to the supernatant. The resultant precipitate is then collected.

The present invention will be described in detail in the following Examples which should not be construed to restrict the invention.

### Example 1. Identification of Bacillus species secreting γ -glutamyl transpeptidase

121 strains of the genus Bacillus were plated on BY agar medium [2% powdered bouillon (Kyokuto Seiyaku Kogyo), 0.5% yeast extract (Daigo Eiyo Kagaku), 1.5% agar, pH 7.2 adjusted with NaOH] and incubated at 30°C overnight. One platinum loop of the culture was transferred to 5 ml of a BYG medium [2% powdered bouillon, 0.5% powdered yeast extract (Daigo Eiyo Kagaku), and 2% glucose, pH 7.2 adjusted with NaOH] and incubated with shaking at 30 °C for 16 hours. 0.25 ml of the culture thus obtained was passaged to 5 ml of a DGYP medium [2% glycerol, 0.3% powdered yeast extract, 2% peptone, 0.05% potassium dihydrogen phosphate, 0.15% dipotassium hydrogen phosphate, 0.05% magnesium sulfate 7 hydrates 0.002% ferrous sulfate 7 hydrate, 0.002% manganese sulfate 4 - 6 hydrate, and 100 µg/liter of thiamine hydrochloride, pH 7.2 adjusted with NaOH] and incubated at 30 °C for 48 hours.

The resultant culture was centrifuged at 3,000 rpm for 15 minutes and the supernatant was tested for γ-glutamyl transpeptidase activity as described hereinafter.

### Method for the determination of γ -glutamyl transpeptidase activity

1 ml of the appropriately diluted supernatant was pre-warmed at 30°C for 2 minutes. To the mixture, 1 ml of the substrate solution [10mM γ -glutamyl-p-nitroanilide/0.2 M Tris-HCl (pH 8.5)] which was pre-warmed to 30 °C was added and the enzymatic reaction was allowed to proceed at 30 °C for 10 minutes. After 10 minutes, the reaction was stopped by adding 2 ml of 20% acetic acid, followed by centrifugation. The supernatant was analyzed for the amount of p-nitroaniline on a colorimeter at 410 nm. As a control, a solution prepared by adding 2 ml of 20% acetic acid, then 1 ml of the substrate solution to 1 ml of the appropriately diluted supernatant was used.

1 unit (U) of γ -glutamyl transpeptidase is defined as the amount of the enzyme which generates 1 µM of p-nitroaniline in 1 minute. The following formula is used for the calculation of the enzyme activity:
γ -glutamyl transpeptidase activity (U) = 0.523 x (E - Eb)x 1/10,
where U is γ -glutamyl transpeptidase activity in 1 ml of the diluted supernatant, E is absorbance of samples and Eb is absorbance of a control at 410 nm.
γ -glutamyl transpeptidase activity (U/ml) of the strains is given in Table 1.

**Table 1**

| Strain | γ-glutamyl transpeptidase activity (U/ml) |
|---|---|
| Bacillus subtilis SJ138 | 1.4 |
| Bacillus subtilis ATCC 9372 | 0.7 |
| Bacillus brevis ATCC 10027 | 0.6 |

### Example 2. Cloning of the γ -glutamyl transpeptidase gene derived from a Bacillus subtilis SJ138 strain

### (1) Purification of γ -glutamyl transpeptidase produced by Bacillus subtilis SJ138

Bacillus subtilis SJ138 was streaked out on BY agar medium and incubated at 30 °C overnight. One platinum loop of the culture was inoculated into 5 ml of BYG medium,and incubated with shaking at 30°C for 6 hours. 0.8 ml of the resultant culture was passaged to 40 ml of BYG medium and incubated with shaking at 30 °C for 10 hours. The mixture was transferred to a 2 ℓ jarfermenter containing 800 ml of GYP medium [8% glycerol, 0.3% yeast extract, 2% peptone, 0.05% potassium hydrogen phosphate, 0.15% dipotassium hydrogen phosphate, 0.05% magnesium sulfate 7 hydrate, 0.002% ferrous sulfate 7 hydrate, 0.002% manganese sulfate 4 - 6 hydrate, and 100 µg/liter thiamine hydrochloride, pH 7.2 adjusted with NaOH] and incubated (700 rpm, 1 vvm) at 30 °C for 20 hours. The culture was centrifuged at 8,000 rpm for 15 minutes, and an equal volume of ethanol was added to the supernatant. The mixture was placed on ice for an hour and then centrifuged at 8000 rpm for 15 minutes. The precipitate formed was suspended in 80 ml of water. 800 mg of lysine monohydrochloride was added to the suspension, and the mixture was lyophilized at -20 °C in vacuo. About 1.4g of the sample thus obtained was resuspended in 50 mM Tris-HCl (TB), pH 7.5 and the mixture was dialyzed against TB. After dialysis, the sample was loaded on a DEAE-cellulose (Serva) column (160 ml capacity) equilibrated with TB. The column was washed with 500 ml of TB, and eluted with 500 ml of TB using a 0 - 0.5M gradient of NaCl. The fractions eluted were analyzed for γ -glutamyl transpeptidase activity using the procedure as described in Example 1. The fractions having γ -glutamyl transpeptidase activity were concentrated using a membrane and the concentrate was dialyzed against 5 mM phosphate buffer, pH 6.8. After dialysis, the sample was loaded on a hydroxyapatite column (60 ml capacity) equilibrated with 5 mM phosphate buffer, pH 6.8, and eluted with 120 ml each of 200 mM, 300 mM, and 400 mM phosphate buffer, pH 6.8. γ -glutamyl transpeptidase activity was found in the 400mM phosphate eluate. This fraction was dialyzed against TB and then concentrated using a membrane. The concentrate was dialyzed further against TB to give purified γ -glutamyl transpeptidase. γ -Glutamyl transpeptidase thus purified was electrophoresed on SDS-polyacrylamide gel to yield two bands of about 41 Kda and 23 Kda, corresponding to a large and small subunit of γ -glutamyl transpeptidase, respectively. No contaminating protein could be observed.

### (2) Amino acid sequencing of γ -glutamyl transpeptidase

Purified γ -glutamyl transpeptidase was loaded on a reverse high pressure liquid chromatography column to separate the large and small subunits. Both subunits were trypsinized according to standard methods. The peptide fragments thus produced were fractionated by reverse high pressure liquid chromatography and analyzed using a 470A Sequencer (Applied Biosystems). The amino acid sequence of one of the trypsinized peptides from each of the large and small subunits is shown below. The amino acid sequence from the large subunit is as defined in the Sequence Listing by SEQ ID No. 3.

The amino acid sequence from the small subunit is as defined in the Sequence Listing by SEQ ID No. 5.

### (3) Synthesis of oligonucleotides corresponding to the amino acid sequence of γ -glutamyl transpeptidase

The oligonucleotides shown below were synthesized using a 380A DNA Synthesizer (Applied Biosystems Corp).
- Probe L:: as defined in the Sequence Listing by SEQ ID No. 4.
- Probe S:: as defined in the Sequence Listing by SEQ ID No. 6.
(wherein N is A, T, G, or C, Y is T or C, and R is A or G).

To 30 ng of the oligonucleotide thus obtained, a mixture of 30 µ Ci γ -³²P-ATP (Amersham), 1 unit of T4 polynucleotide kinase (Takara Shuzo), and 3 µ 1 of a kinase buffer (0.5M Tris-HCl, 100 mM MgCl₂, and 100 mM dithiothreitol, (pH 7.5)) was was added. The resultant mixture (30 µ l) was incubated at 37 °C for 30 minutes to label the 5' end of the oligomer with ³²P. Then, 10 µ g of salmon sperm DNA was added and the mixture was extracted with phenol. The extract was loaded on a DEAE Sephadex G-50 (Pharmacia) column equilibrated with 20 mM Tris-HCl (pH 7.5)/100 mM NaCl/1 mM EDTA and eluted to give the labelled oligomer.

### (4) Southern hybridization of a chromosomal γ -gutamyl transpeptidase gene of SJ138 using probes L and S

A chromosomal γ -glutamyl transpeptidase gene of SJ138 was detected by Southern hybridization method using probes L and S, as described herein-after.

40 ml of BYG culture medium was inoculated with bacteria of the strain SJ138 and incubated at 30 °C overnight. From the culture, the chromosomal DNA was prepared according to the method described by Saito and Miura [H. Saito and K. Miura: Biochim. Biophys. Acta, 72, 619 (1963)]. 2 µg of the chromosomal DNA was digested with EcoRI and HindIII, and the digests were electrophoresed together with a molecular weight marker λ DNA (Takara Shuzo) on three sets of agarose gels. One of the sets was stained with ethidium bromide and then photographed beside a scale for the comparison of mobility. The rest of the gels was consecutively immersed in a 10-fold volume each of 0.5N NaOH/1M NaCl, and 0.5M Tris-HCl (pH 7.5)/2.5M NaCl for 30 minutes to allow denaturation. A nitrocellulose filter was placed on each of the gels. The filter/gel was placed in the tray filled with 20 X SSC [3M sodium chloride and 0.3M trisodium citrate, (pH 7.0)]. DNA was allowed to transfer to the nitrocellulose filters for 16 hours. Then, the filters were air-dried and baked at 80 °C for two hours.

Two filters thus treated were placed in 6 ml of pre-hybridization solution [0.02% Fycoll, 0.02% bovine serum albumin, 0.02% polyvinyl pyrrolidone, 0.9M sodium chloride, 0.09M trisodium citrate, 50 mM Tris-HCl (pH 7.0), and 50 µg/ml of heat denatured salmon sperm DNA] in a vinyl bag and pre-hybridized at 65 °C for 3 hours. After pre-hybridization, the filters were removed from the bag and placed in a separate bag. 3 ml of fresh pre-hybridization solution was then added to each bag, and 2 µ Ci each of the ³²P-labelled probes L and S which had previously been heated at 95 °C for 5 minutes was added separately to one of the bags. The bags were incubated at 34 °C for 16 hours. After hybridization, the filter was removed from each bag and washed with 3 × 100 ml of 6 × SSC [0.9M sodium chloride and 0.09M trisodium citrate (pH 7.0)] at 20 °C for 10 minutes. The filters were air-dried and exposed to a X-ray film at -80 °C for two days. The film was developed and the bands that appeared on the film were compared with those on the photograph of the gel. It has been found that the probes L and S hybridized to the 2.9 Kb EcoRI fragment and the 2.8 Kb HindIII fragment.

### (5) Cloning of γ -glutamyl transpeptidase encoding DNA using Bacillus subtilis host-vector system.

Since the possibility that the nucleotide sequence encoding the two subunits of γ -glutamyl transpeptidase can be cloned as a single insert was shown from the results described above, a "shot gun cloning" was carried out using a Bacillus subtilis host-vector system. Bacteria of the strain Bacillus subtilis Marburg BR151 were first transformed with pUB110 derived from Bacillus subtilis. The transformant was designated BR151/pUB110 and used as a host. pBD64 [D. Dubnau et al., J. Bacteriol., 141, 246 (1980)] derived from Bacillus subtilis was digested with BamHI and ligated with chromosomal DNA of strain SJ138 partially digested with Sau3A, using T4 DNA ligase (Takara Shuzo). The construct was used to transform the strain BR151/pUB110. The transformants were plated on the nitrocellulose filter on a GT agar medium [0.2% maltose, 0.25% monosodium glutamate, 50 µ g/ml of tryptophan, 0.05% potassium dihydrogen phosphate, 0.15% dipotassium hydrogen phosphate, 0.05% magnesium sulfate 7 hydrate, 0.002% ferrous sulfate 7 hydrate, 0.002% manganese sulfate 4-6 hydrate, 100 µ g/liter of thiamine hydrochloride, and 1.5% agar, (pH 7.2 adjusted with NaOH)] containing 5 µ g/ml of chloramphenicol (Cm), and incubated at 30 °C for three days. After the colonies appeared on the filter, the filter was placed on a filter pre-soaked with 5mM γ -glutamyl-p-nitroanilide/0.2M Tris-HCl (pH 8.5), and incubated at 37 °C . Certain colonies showed a yellow pigment around them, suggesting that γ -nitroaniline was formed by catalytic action of γ -glutamyl transpeptidase from the substrate γ -glutamyl-p-nitroanilide.

A plasmid designated pG31 was obtained from one of those colonies according to the method described by Gryczan et al. [T. J. Gryczan et al., J. Bacteriol., 134, 318 (1978)]. Southern hybridization of pG31 was carried out using the ³²P-labelled probes L and S according to the method described in Examples 2(3) and (4). The results demonstrated that certain DNA sequence on pG31 hybridized to both probes, suggesting that pG31 contained the cloned nucleotide sequences of the large and small subunit of γ -glutamyl transpeptidase gene.

Southern hybridization of chromosomal DNA of SJ138 was carried out using the 1.0 Kb EcoRI fragment of pG31 as a probe, in order to determine the source of the cloned insert in pG31. 5 µ g of pG31 DNA was digested with EcoRI and electrophoresed on an agarose gel. The 1.0 Kb EcoRI band was excised from the gel and placed in a dialysis tube. 0.3 ml of Tris-borate (90mM Tris-borate and 4mM EDTA, pH 8.3) was added to the tube, and the tube was sealed and placed in the vessel filled with the same Tris-borate. Electroelution was carried out at 50 mA for 10 min. Then, the current was reversed for 20 seconds to detach DNA from the dialysis tube. The DNA suspension was removed from the tube. 1/10 volume of 3M sodium acetate, pH 5.6 and 3 volumes of ice cold ethanol were added to the DNA suspension. The mixture was centrifuged and the pelleted DNA was recovered. The DNA was dried and resuspended in 10 µ 1 of TE buffer (20mM Tris-HCl, 1mM EDTA, pH 7.5)

DNA thus obtained was radiolabelled according to the nick translation method [P. W. J. Rigby et al., J. Mol. Biol., 113, 237 (1977)]. Southern hybridization of BglII digests of of chromosomal DNAs of SJ138 and Bacillus subtilis Marburg BR151 was carried out using the above labelled fragment as a probe. A 9.5 Kb BglII fragment of chromosomal DNA of SJ138 showed a high degree of hybridization, while that of BR151 showed a poor degree of hybridization. The results demonstrated that the DNA insert in pG31 was derived from chromosomal DNA of the SJ138 strain.

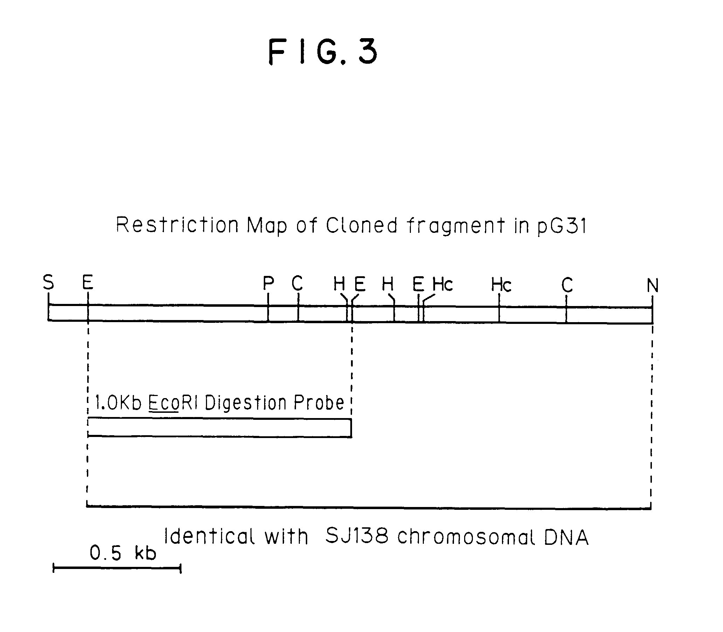

Furthermore, Southern hybridization of pG31 DNA and SJ138 chromosomal DNA which were digested with various restriction enzymes was carried out using the above labelled 1.0 Kb EcoRI fragment as a probe. It has been revealed that the 2.1 Kbf the EcoRI-NruI region on the chromosomal DNA of SJ138 are identical with that of the cloned DNA fragment in pG31 (See Fig. 3).

### Example 3. Determination of DNA sequence of the γ-glutamyl transpeptidase gene

To determine the nucleotide sequence of the γ - glutamyl transpeptidase gene, pG31 was digested with various restriction enzymes, and the restriction fragments were cloned into pUC118 and pUC119. The sizes of the fragments were reduced using an exonuclease III deletion kit (Takara Shuzo), if necessary.

The constructs thus obtained were used for the transformation of MV1184, an E. coli K12 substrain. The transformant was cultured in a 2X TY medium (1.6% bactotrypton, 1% yeast extract, and 0.5% NaCl, pH 7.4) containing 100 µ g/ml of Ampicillin at 37 °C overnight. 30 µ l of the overnight culture was added to 3 ml of 2X TY medium containing 100 µ g/ml of Ampicillin, and incubated at 37 °C for three hours. Then helper phage M13K07 was added to the mixture at a M.O.I.=3. At the same time, Kanamycin (Km) was added to the mixture to a final concentration of 70 µ g/ml, and the mixture was incubated further at 37 °C overnight. Single-stranded DNA was prepared from the phage particle [J. Vieira and J. Messing: Methods in Enzymology, 153, 3, Academic Press (1987)] and used for DNA sequencing using the dideoxy chain termination method. 2382 bases of the nucleotide sequence from the SphI to the NruI restriction site, determined as above, are as defined in the Sequence Listing by SEQ ID No. 2.
-35 and -10: presumed promoter region. RBS: presumed ribosome binding site. →← : presumed transcription termination sequence.

An open reading frame 1 (ORF1) encoding 591 amino acids has been found within the nucleotide sequence between the SPhI andNruI recognition sites. A complementary sequence AAAGGAGG to that of the 3' end of the Bacillus subtilis 16S ribosomal RNA has been found at the upstream region in the ORF1 and the sequence is believed to be a ribosome binding site. It has also been found that the upstream region of a ribosome binding site contains the presumed -35 sequence (AAAACA) and -10 sequence (TATAAT). Furthermore, a potential stem loop structure as defined in the Sequence Listing of SEQ ID No.9, which is believed to be involved in the transcriptional termination, has been found at the downstream region in the ORF1. However, the nucleotide sequence controlling the gene expression has not been found within the ORF1.

Amino acids 1-30 of the ORF1 show a typical signal sequence found in the genus Bacillus (Yamane, Nippon Nogeikagakukai-shi, 61, 64, 1987), and is the only signal sequence found in the ORF1. The sequence of amino acids 378 to 390 is identical with the partial amino acid sequence of the large subunit, and the amino acid sequence from 503 to 510 is identical with the partial sequence of the small subunit. Taken together, these findings indicate that the γ -glutamyl transpeptidase gene of Bacillus subtilis SJ138 strain encodes both the large and small subunits of γ -glutamyl transpeptidase as a single polypeptide.

234 bases starting from a SphI recognition site on the nucleotide sequence of pG31 are identical with 235 bases of the nucleotide numbers 789-1023 of pUB110 [T. Mckenzie et al., Plasmid, 15, 93 (1986)] except for a G at the nucleotide number 860. The nucleotide numbers 230-234 of pG31 representing (part of) a BamHI and Sau3A recognition site are believed to be one of the ligation sites. An EcoRI site is located in the 40 nucleotides downstream of said ligation site of pG31. A NruI site is located 2142 nucleotides downstream of the ligation site. The site 14 nucleotides away from the Nrul site on pG31 is believed to be the other ligation site, a Sau3A/BamHI site, and the sequence following the site is the same sequence as found upstream of the nucleotide number 788 in pUB110.

The results described above and in Example 2 (5) demonstrate that the 2382 bases long sequence between a SphI and NruI recognition site on pG31 is solely derived from the nucleotide sequence of pUB110 and SJ138 chromosomal DNA. The self-cloning of the γ -glutamyl transpeptidase gene is achieved by cloning the 2382 bases long sequence into a plasmid vector derived from Bacillus subtilis.

### Example 4. Construction of a transformant containing multiple copies of the γ -glutamyl transpeptidase gene

To carry out self-cloning, the SphI-NruI fragment of pG31 containing the gene was inserted into the vector pEX653, a derivative of Bacillus subtilis pUB110. The vector pEX653 was constructed as shown in Fig. 2 and described below. 5 µ g of Bacillus subtilis plasmid pUB110 DNA and 5 µ g of E. coli plasmid pUC19 DNA (Takara Shuzo) were digested with EcoRI and XbaI and ligated using T4 DNA ligase. The resultant construct was used to transform Bacillus subtilis Marburg BR151. The transformant was screened on BY agar medium containing 5 µ g/ml of Kanamycin (Km). Plasmid DNA of Km resistant transformants was prepared according to the method described in Example 2 (5). A plasmid pEX653 thus obtained was confirmed to contain a partial multiple linker site, EcoRI-SacI-KpnI-SmaI-BamHI-XbaI, derived from pUC19 in place of the EcoRI-XbaI fragment of pUB110.

The subcloning of the γ -glutamyl transpeptidase gene into pEX653 was carried out as follows. 5 µ g of pEX653 DNA was digested with SphI and SmaI, extracted with phenol, and precipitated with ethanol to recover DNA fragments. In the meantime, 5 µ g of pG31 DNA was digested with SphI and NruI, extracted with phenol and precipitated with ethanol. Both pEX653 and pG31 restriction fragments were suspended in TE buffer separately, mixed and ligated using T4 DNA ligase. The construct was used to transform Bacillus subtilis Marburg BR151. The transformants were screened on the nitrocellulose filter on GT agar medium containing 5 µ g/ml of Km according to the method described in Example 2 (5). A colony showing γ -glutamyl traspeptidase activity was picked and cultured. Plasmid DNA was prepared according to the method described in 2 (5), digested with restriction enzymes and electrophoresed on an agarose gel for analysis.

One of the plasmids, designated pUG55, contains a SphI-NruI fragment of about 2.4 Kb encoding the γ -glutamyl transpeptidase at the SphI-SmaI site of pEX653 (See Fig. 2).

pUG55 was introduced into protoplastsof Bacillus sublitis SJ138, a γ -glutamyl transpeptidase producing strain according to the method described by Akamatsu and Sekiguchi [T. Akamatsu and J. Sekiguchi: Agric. Biol. Chem., 46, 1617 (1982)]. The transformants were screened on a minimum essential medium containing 100 µg/ml of Km. The colony grown on the plate was picked and cultured. Plasmid DNA was prepared as described in Example 2 (5), digested with restriction enzymes, electrophoresed on agarose gels and analyzed. The plasmid DNA as prepared above was found to be identical with the plasmid DNA used for transformation.

### Example 5. Production of γ -glutamyl transpeptidase using various transformants

The γ -glutamyl transpeptidase production of various strains was tested: Bacillus subtilis Marburg BR151 alone, BR151 containing pG31, BR151 containing pEX653, BR151 containing pUG55, Bacillus subtilis SJ138 alone, SJ138 containing pEX653, SJ138 containing pUG55. Each strain was plated on BY agar medium and incubated at 30 °C overnight. One platium loop of the culture was transferred to 3ml of BYG medium and incubated at 30 °C overnight. 0.25 ml of the resultant culture was added to 5 ml of GYP medium in a tube. The mixture was incubated at 30 °C for 72 hours. In the case of the strains containing a plasmid, Kanamycin was added to both the BYG and GYP media to a final concentration of 5 µ g/ml. After incubation, the culture was centrifuged to remove the bacterial cells and the supernatant was tested for γ -glutamyl transpeptidase activity colorimetrically as described in Example 1 . The γ - glutamyl transpeptidase production of various strains is given in Table 2.

**Table 2**

| Strain | γ-Glutamyl Transpeptidase |
|---|---|
| | Activity (U/ml) |
| Bacillus subtilis Marburg | |
| BR151 | 0 |
| BR151/pG31 | 45.6 |
| BR151/pEX653 | 0 |
| BR151/pUG55 | 48.5 |
| Bacillus subtilis | |
| SJ138 (FERM BP-2694) | 3.0 |
| SJ138/pEX653 | 2.8 |
| SJ138/pUG55 | 73.5 |
| (SJ139, FERM BP-2695) | |

The culture was incubated in BYG(3 ml) medium at 30°C overnight. The culture in BYG(0.25 ml) was incubated in GYP (5 ml) medium at 30°C for 72 hr.

Introduction of a recombinant plasmid containing a γ -glutamyl transpeptidase gene into a γ -glutamyl transpeptidase non-producing or producing strain causes a production or an increase in γ -glutamyl transpeptidase production, respectively, as shown in Table 2.

### INFORMATION FOR SEQ ID NO:1:

(i) SEQUENCE CHARACTERISTICS:
   (A) LENGTH: 1773 bases
   (B) TYPE: Nucleic acid
   (C) STRANDEDNESS: Single
   (D) TOPOLOGY: Linear
(ii) MOLECULE TYPE: Genomic DNA
(vi) ORIGINAL SOURCE
   (A) ORGANISM: Bacillus subtilis
   (B) STRAIN: SJ138 (FERM BP-2694)
(xi) SEQUENCE DESCRIPTION: SEQ ID NO:1:

10 20 30 40 50 60
ATGAAAAAGA AAAAGTTTAT GAATCTCTGT TTTATCGTTC TGCTCAGTAC TTTGCTCGCG

70 80 90 100 110 120
GCCGGAAGCA TCCCTTATCA CGCTCAGGCT AAGAAACACC CGTTTTCCTA TGACGACTAC

130 140 150 160 170 180
AAACAGGTAG ATGTCGGCAA AGACGGCATG GTTGCCACCG CCCATCCTCT CGCTTCACAA

190 200 210 220 230 240
ATCGGTGCCG ACGTGCTGAA AAAAGGCGGC AATGCAATTG ACGCCGCGGT TGCCATTCAA

250 260 270 280 290 300
TTCGCATTAA ACGTAACTGA ACCTATGATG TCTGGAATCG GCGGCGGCGG ATTTATGATG

310 320 330 340 350 360
GTTTATGATG CGAAGACGAA AGACACCACC ATTATCGACA GCAGGGAACG CGCACCGGCA

370 380 390 400 410 420
GGCGCAACAC CGGATATGTT CCTTGACGAA AACGGCAAAG CCATTCCTTT CTCCGAACGC

430 440 450 460 470 480
GTTACGAAAG GGACTGCAGT CGGTGTTCCG GGAACATTAA AAGGACTTGA AAAAGCGCTA

490 500 510 520 530 540
GACAAATGGG GCACACGCTC CATGAAACAA CTCATCACCC CTTCCATTAA ACTTGCCTCA

550 560 570 580 590 600
AAAGGCTTTC CGATCGATTC GGTTTTAGCT GATGCCATCT CAGATTATAA AGACAAATTA

610 620 630 640 650 660
TCACACACTG CTGCAAAAGA CGTGTTTCTT CCGGACGGAG AACCTCTGAA AGAAGGAGAC

670 680 690 700 710 720
ACACTCATCC AAAAAGACTT AGCCAAAACA TTTACAGCTA TTAAGTACAA AGGCACAAAA

730 740 750 760 770 780
GCATTCTATG ACGGTGCATT CTCCAAAAAG CTTGCAGAAA CAGTGCAGGA ATTCGGCGGC

790 800 810 820 830 840
TCAATGACAG AAAAAGACAT TAAAAACTTC AATGTGACGA TTGACGAACC GATCTGGGGA

850 860 870 880 890 900
GATTACCAGG GCTATCATAT CGCAACTGCT CCTCCTCCAA GCTCGGGCGG TGTTTTCCTG

910 920 930 940 950 960
TTGCAAATGC TGAACCTCCT GGATGATTTT AAGCTTTCTC AATATGATAT CCGTTCTTGG

970 980 990 1000 1010 1020
CAAAAATATC AGCTTCTCGC AGAAACGATG CATTTGGCTT ATGCTGACCG CGCCGCATTT

1030 1040 1050 1060 1070 1080
GCCGGGGACC CAGAATTCGT CAACGTCCCT CTCAAAGGTC TCTTGAATCC AGATTATATC

1090 1100 1110 1120 1130 1140
AATGCCCGCA GACAGCTGAT AGATATTGAT AAAGTCAATA AAAAACCGAA AGCCGGCGAT

1150 1160 1170 1180 1190 1200
CCTTGGGCCT ATCAGGAAGG TTCTGCAAAC TATAAACAAG TGGAGCAGCC GACTGACAAA

1210 1220 1230 1240 1250 1260
CAAGAAGGTC AAACGACTCA CTTCACGGTA ACCGACCGCT TCGGCAATGT CGTATCTTAT

1270 1280 1290 1300 1310 1320
ACGACAACAA TTGAACAGCT GTTCGGTTCC GGCATTATGG TTCCCGGATA CGGCGTTGTG

1330 1340 1350 1360 1370 1380
CTGAATAATG AGTTAACAGA CTTCGATGCG GTGCCTGGCG GCGCAAATGA AGTGCAGCCG

1390 1400 1410 1420 1430 1440
AATAAACGTC CGCTCAGCAG CATGACTCCG ACTATTTTAT TCAAAAATAA CGAACCTGTC

1450 1460 1470 1480 1490 1500
CTGACTGTCG GCTCCCCCGG CGGAGCAACG ATTATTTCTT CCGTCCTGCA AACGATCCTG

1510 1520 1530 1540 1550 1560
AACAAAGTTG AGTACGGCAT GGATCTGAAA GCGGCAGTCG AAGAGCCGAG AATTTACACA

1570 1580 1590 1600 1610 1620
AACAGCATGA CATCCTATCG ATATGAAAAA GGAGTGCCGG AAGAAGCCCG CACAAAACTG

1630 1640 1650 1660 1670 1680
AACGAAATGG GGCATAAATT CGGCAGCAGC CCGGTTGATA TCGGTAATGT GCAAAGCATC

1690 1700 1710 1720 1730 1740
CTGATCGACC GTGAAAACGG CACCTTCACC GGAGTCGCCG ACTCAAGCCG AAACGGAGCC

1750 1760 1770
GCAATCGGCG TAAACTTGAA AAATTATAAA AAA

### INFORMATION FOR SEQ ID NO: 2

(i) SEQUENCE CHARACTERISTICS:
   (A) LENGTH: 2382 bases
   (B) TYPE: Nucleic acid
   (C) STRANDEDNESS: Single
   (D) TOPOLOGY: Linear
(ii) MOLECULE TYPE: Genomic DNA
(vi) ORIGINAL SOURCE:
   (A) ORGANISM: Bacillus subtilis
   (B) STRAIN: SJ138 (FERM BP-2694)
(ix) FEATURE:
   430-435 E-35 signal
   453-458 E-10 signal
   473-480 RBS
   487-2259 Smooth peptide
(xi) SEQUENCE DESCRIPTION: SEQ ID NO:2

GCATGC
SphI
AATTTCATAATCAAAGAGAGCGAAAAAGTAGAACGAATGATGATATTGACCATGAGCGAA
CACGTGAAAATTATGATTTGAAAAATGATAAAAATATTGATTACAACGAACGTGTCAAAG
AAATTATTGAATCACAAAAAACAGGTACAAGAAAAACGAGGAAAGATGCTGTTCTTGTAA
ATGAGTTGCTAGTAACATCTGACCGAGATTTTTTTGAGCAACTGGATCGTTAAATACTGG
CTCACGTTCCTTCATATGTCATGTGAGTGAATTCTGTTTTGTTTATTGTAGCTTATTTTT
TGTCTTTATGCTTGTTTCACAGCTTTTTCAGTCCGGTTTCCCATTTAGCCTATTTGCGAC
TGATTACATTCACACAGAAACCCCAACTTTTTGCACACCGGACTATTCCGTTTGTCACTT
GTGAAAACAGCACATTTTACTTACTCTATAATTGTAAGCGGAAAACAAAGGAGGCAGACT
-35 -10 RBS
10 20 30 40 50 60
ATGAAAAAGAAAAAGTTTATGAATCTCTGTTTTATCGTTCTGCTCAGTACTTTGCTCGCG
MetLysLysLysLysPheMetAsnLeuCysPheIleValLeuLeuSerThrLeuLeuAla

70 80 90 100 110 120
GCCGGAAGCATCCCTTATCACGCTCAGGCTAAGAAACACCCGTTTTCCTATGACGACTAC
AlaGlySerIleProTyrHisAlaGlnAlaLysLysHisProPheSerTyrAspAspTyr

130 140 150 160 170 180
AAACAGGTAGATGTCGGCAAAGACGGCATGGTTGCCACCGCCCATCCTCTCGCTTCACAA
LysGlnValAspValGlyLysAspGlyMetValAlaThrAlaHisProLeuAlaSerGln

190 200 210 220 230 240
ATCGGTGCCGACGTGCTGAAAAAAGGCGGCAATGCAATTGACGCCGCGGTTGCCATTCAA
IleGlyAlaAspValLeuLysLysGlyGlyAsnAlaIleAspAlaAlaValAlaIleGln

250 260 270 280 290 300
TTCGCATTAAACGTAACTGAACCTATGATGTCTGGAATCGGCGGCGGCGGATTTATGATG
PheAlaLeuAsnValThrGluProMetMetSerGlyIleGlyGlyGlyGlyPheMetMet

310 320 330 340 350 360
GTTTATGATGCGAAGACGAAAGACACCACCATTATCGACAGCAGGGAACGCGCACCGGCA
ValTyrAspAlaLysThrLysAspThrThrIleIleAspSerArgGluArgAlaProAla

370 380 390 400 410 420
GGCGCAACACCGGATATGTTCCTTGACGAAAACGGCAAAGCCATTCCTTTCTCCGAACGC
GlyAlaThrProAspMetPheLeuAspGluAsnGlyLysAlaIleProPheSerGluArg

430 440 450 460 470 480
GTTACGAAAGGGACTGCAGTCGGTGTTCCGGGAACATTAAAAGGACTTGAAAAAGCGCTA
ValThrLysGlyThrAlaValGlyValProGlyThrLeuLysGlyLeuGluLysAlaLeu

490 500 510 520 530 540
GACAAATGGGGCACACGCTCCATGAAACAACTCATCACCCCTTCCATTAAACTTGCCTCA
AspLysTrpGlyThrArgSerMetLysGlnLeuIleThrProSerIleLysLeuAlaSer

550 560 570 580 590 600
AAAGGCTTTCCGATCGATTCGGTTTTAGCTGATGCCATCTCAGATTATAAAGACAAATTA
LysGlyPheProIleAspSerValLeuAlaAspAlaIleSerAspTyrLysAspLysLeu

610 620 630 640 650 660
TCACACACTGCTGCAAAAGACGTGTTTCTTCCGGACGGAGAACCTCTGAAAGAAGGAGAC
SerHisThrAlaAlaLysAspValPheLeuProAspGlyGluProLeuLysGluGlyAsp

670 680 690 700 710 720
ACACTCATCCAAAAAGACTTAGCCAAAACATTTACAGCTATTAAGTACAAAGGCACAAAA
ThrLeuIleGlnLysAspLeuAlaLysThrPheThrAlaIleLysTyrLysGlyThrLys

730 740 750 760 770 780
GCATTCTATGACGGTGCATTCTCCAAAAAGCTTGCAGAAACAGTGCAGGAATTCGGCGGC
AlaPheTyrAspGlyAlaPheSerLysLysLeuAlaGluThrValGlnGluPheGlyGly

790 800 810 820 830 840
TCAATGACAGAAAAAGACATTAAAAACTTCAATGTGACGATTGACGAACCGATCTGGGGA
SerMetThrGluLysAspIleLysAsnPheAsnValThrIleAspGluProIleTrpGly

850 860 870 880 890 900
GATTACCAGGGCTATCATATCGCAACTGCTCCTCCTCCAAGCTCGGGCGGTGTTTTCCTG
AspTyrGlnGlyTyrHisIleAlaThrAlaProProProSerSerGlyGlyValPheLeu

910 920 930 940 950 960
TTGCAAATGCTGAACCTCCTGGATGATTTTAAGCTTTCTCAATATGATATCCGTTCTTGG
LeuGlnMetLeuAsnLeuLeuAspAspPheLysLeuSerGlnTyrAspIleArgSerTrp

970 980 990 1000 1010 1020
CAAAAATATCAGCTTCTCGCAGAAACGATGCATTTGGCTTATGCTGACCGCGCCGCATTT
GlnLysTyrGlnLeuLeuAlaGluThrMetHisLeuAlaTyrAlaAspArgAlaAlaPhe

1030 1040 1050 1060 1070 1080
GCCGGGGACCCAGAATTCGTCAACGTCCCTCTCAAAGGTCTCTTGAATCCAGATTATATC
AlaGlyAspProGluPheValAsnValProLeuLysGlyLeuLeuAsnProAspTyrIle

1090 1100 1110 1120 1130 1140
AATGCCCGCAGACAGCTGATAGATATTGATAAAGTCAATAAAAAACCGAAAGCCGGCGAT
AsnAlaArgArgGlnLeuIleAspIleAspLysValAsnLysLysProLysAlaGlyAsp

1150 1160 1170 1180 1190 1200
CCTTGGGCCTATCAGGAAGGTTCTGCAAACTATAAACAAGTGGAGCAGCCGACTGACAAA
ProTrpAlaTyrGlnGluGlySerAlaAsnTyrLysGlnValGluGlnProThrAspLys
Corres. to probe L
1210 1220 1230 1240 1250 1260
CAAGAAGGTCAAACGACTCACTTCACGGTAACCGACCGCTTCGGCAATGTCGTATCTTAT
GlnGluGlyGlnThrThrHisPheThrValThrAspArgPheGlyAsnValValSerTyr

1270 1280 1290 1300 1310 1320
ACGACAACAATTGAACAGCTGTTCGGTTCCGGCATTATGGTTCCCGGATACGGCGTTGTG
ThrThrThrIleGluGlnLeuPheGlySerGlyIleMetValProGlyTyrGlyValVal

1330 1340 1350 1360 1370 1380
CTGAATAATGAGTTAACAGACTTCGATGCGGTGCCTGGCGGCGCAAATGAAGTGCAGCCG
LeuAsnAsnGluLeuThrAspPheAspAlaValProGlyGlyAlaAsnGluValGlnPro

1390 1400 1410 1420 1430 1440
AATAAACGTCCGCTCAGCAGCATGACTCCGACTATTTTATTCAAAAATAACGAACCTGTC
AsnLysArgProLeuSerSerMetThrProThrIleLeuPheLysAsnAsnGluProVal

1450 1460 1470 1480 1490 1500
CTGACTGTCGGCTCCCCCGGCGGAGCAACGATTATTTCTTCCGTCCTGCAAACGATCCTG
LeuThrValGlySerProGlyGlyAlaThrIleIleSerSerValLeuGlnThrIleLeu

1510 1520 1530 1540 1550 1560
AACAAAGTTGAGTACGGCATGGATCTGAAAGCGGCAGTCGAAGAGCCGAGAATTTACACA
AsnLysValGluTyrGlyMetAspLeuLysAlaAlaValGluGluProArgIleTyrThr
Corres. to probe S
1570 1580 1590 1600 1610 1620
AACAGCATGACATCCTATCGATATGAAAAAGGAGTGCCGGAAGAAGCCCGCACAAAACTG
AsnSerMetThrSerTyrArgTyrGluLysGlyValProGluGluAlaArgThrLysLeu

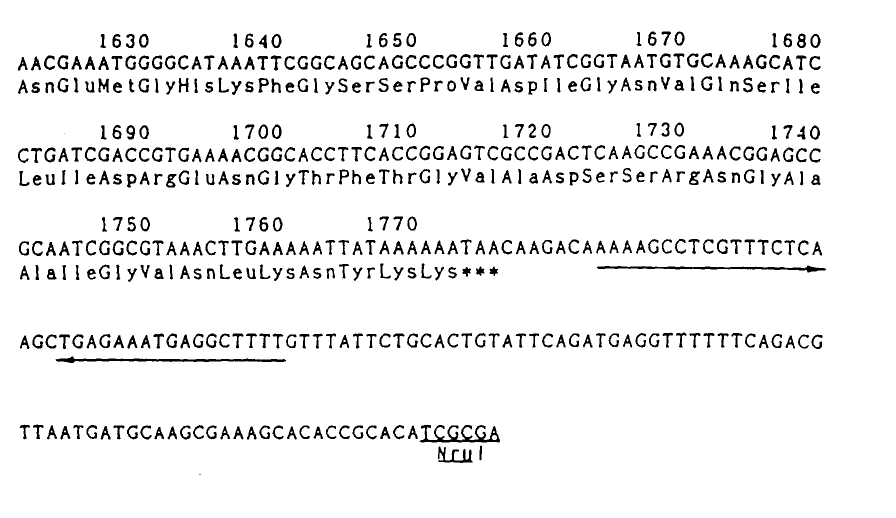
1630 1640 1650 1660 1670 1680
AACGAAATGGGGCATAAATTCGGCAGCAGCCCGGTTGATATCGGTAATGTGCAAAGCATC
AsnGluMetGlyHisLysPheGlySerSerProValAspIleGlyAsnValGlnSerIle

1690 1700 1710 1720 1730 1740
CTGATCGACCGTGAAAACGGCACCTTCACCGGAGTCGCCGACTCAAGCCGAAACGGAGCC
LeuIleAspArgGluAsnGlyThrPheThrGlyValAlaAspSerSerArgAsnGlyAla

1750 1760 1770
GCAATCGGCGTAAACTTGAAAAATTATAAAAAATAACAAGACAAAAGCCTCGTTTCTCA
AlaIleGlyValAsnLeuLysAsnTyrLysLys***

AGCTGAGAAATGAGGCTTTTGTTTATTCTGCACTGTATTCAGATGAGGTTTTTCAGACG

TTAATGATGCAAGCGAAAGCACACCGCACATCGCGA
NruI

### INFORMATION FOR SEQ ID NO : 3

(i) SEQUENCE CHARACTERISTICS:
   (A) LENGTH: 13 amino acids
   (B) TYPE: Amino acid
   (C) TOPOLOGY: Linear
(ii) MOLECULE TYPE: Peptide
(ix) SEQUENCE DESCRIPTION: SEQ ID NO:3
   Ala-Gly-Asp-Pro-Trp-Ala-Tyr-Gln-Glu-Gly-Ser-Ala-Asn

### INFORMATION FOR SEQ ID NO:4

(i) SEQUENCE CHARACTERISTICS:
   (A) LENGTH: 17 bases
   (B) TYPE: Nucleic acid
   (C) STRANDEDNESS: Single
   (D) TOPOLOGY: Linear
(ii) MOLECULE TYPE: Other nucleic acid
(xi) SEQUENCE DESCRIPTION: SEQ ID NO:4
   5'-TGGGCNTAYCARGARGG-3'

### INFORMATION FOR SEQ ID NO:5

(i) SEQUENCE CHARACTERISTICS:
   (A) LENGTH: 8 amino acids
   (B) TYPE: Amino acid
   (C) TOPOLOGY: Linear
(ii) MOLECULE TYPE: Peptide
(xi) SEQUENCE DESCRIPTION: SEQ ID NO:5
   Val-Glu-Tyr-Gly-Met-Asp-Leu-Lys

### INFORMATION FOR SEQ ID NO:6

(i) SEQUENCE CHARACTERISTICS:
   (A) LENGTH: 17 bases
   (B) TYPE: Nucleic acid
   (C) STRANDEDNESS: Single
   (D) TOPOLOGY: Linear
(ii) MOLECULE TYPE: Other nucleic acid
(xi) SEQUENCE DESCRIPTION: SEQ ID NO:6
   5'-GARTAYGGNATGGAYYT-3'

### INFORMATION FOR SEQ ID NO:7

(i) SEQUENCE CHARACTERISTICS:
   (A) LENGTH: 17 bases
   (B) TYPE: Nucleic acid
   (C) STRANDEDNESS: Single
   (D) TOPOLOGY: Linear
(ii) MOLECULE TYPE: Other nucleic acid
(xi) SEQUENCE DESCRIPTION: SEQ ID NO:7
   5'-ARRTCCATNCCRTAYTC-3'

### INFORMATION FOR SEQ ID NO:8

(i) SEQUENCE CHARACTERISTICS:
   (A) LENGTH: 54 bases
   (B) TYPE: Nucleic acid
   (C) STRANDEDNESS: Double
   (D) TOPOLOGY: Linear
(xi) SEQUENCE DESCRIPTION: SEQ ID NO:8
   5'-CTAGAGGATCCCCGGGTACCGAGCTCG- 3'
   5'-TCCTAGGGGCCCATGGCTCGAGCTTAA-5'

### INFORMATION FOR SEQ ID NO:9

(i) SEQUENCE CHARACTERISTICS:
(A) LENGTH: 37 bases
(B) TYPE: Nucleic acid

## Claims

1. A γ-glutamyl transpeptidase gene having the DNA sequence depicted in the attached sequence listing SEQ ID:NO. 1.

2. A recombinant DNA molecule comprising a vector DNA and a DNA fragment containing the γ-glutamyl transpeptidase gene having the DNA sequence depicted in the attached sequence listing SEQ ID: No. 1.

3. A microorganism carrying the recombinant DNA molecule comprising a vector DNA and a DNA fragment containing the γ-glutamyl transpeptidase gene having the DNA sequence depicted in the attached sequence listing SEQ ID: No. 1.

4. The microorganism according to claim 3 which is a microorganism of the genus Bacillus.

5. The microorganism according to claim 4 which is Bacillus subtilis SJ139 (FERM BP-2695).

6. A process for producing γ-glutamyl transpeptidase which comprises
(a) culturing a microorganism of the genus Bacillus carrying the recombinant DNA molecule. comprising a vector DNA and a DNA fragment containing the γ-glutamyl transpeptidase gene having the DNA sequence depicted in the attached sequence listing SEQ ID: No. 1 in a suitable culture medium;
(b) accumulating γ-glutamyl transpeptidase in the culture medium; and
(c) recovering said γ-glutamyl transpeptidase from said culture medium.

## Patentansprüche

1. γ-Glutamyl-Transpeptidase-Gen, das die in dem beiliegenden Sequenzprotokoll als SEQ ID NO: 1 dargestellte DNA-Sequenz hat.

2. Rekombinantes DNA-Molekül, umfassend eine Vektor-DNA und ein DNA-Fragment, das das γ -Glutamyl-Transpeptidase-Gen, das die in dem beiliegenden Sequenzprotokoll als SEQ ID NO: 1 dargestellte DNA-Sequenz hat, enthält.

3. Mikroorganismus, der das rekombinante DNA-Molekül, umfassend eine Vektor-DNA und ein DNA-Fragment, das das γ -Glutamyl-Transpeptidase-Gen, das die in dem beiliegenden Sequenzprotokoll als SEQ ID NO: 1 dargestellte DNA-Sequenz hat, trägt.

4. Mirkroorganismus nach Anspruch 3, der ein Mikroorganismus der Gattung Bacillus ist.

5. Mikroorganismus nach Anspruch 4, der Bacillus subtilis SJ139 (FERM BP-2695) ist.

6. Verfahren zur Herstellung von γ -Gluamyl-Transpeptidase, das umfaßt
(a) Züchten in einem geeigneten Kulturmedium, eines Mikroorganismus der Gattung Bacillus, der das rekombinante DNA-Molekül, umfassend eine Vektor-DNA und ein DNA-Fragment, das das γ-Glutamyl-Transpeptidase-Gen, das die in dem beiliegenden Sequenzprotokoll als SEQ ID NO: 1 dargestellte DNA-Sequenz hat, beinhaltet;
(b) Ansammeln der γ -Glutamyl-Transpeptidase in dem Kulturmedium; und
(c) Gewinnen der γ -Glutamyl-Transpeptidase von dem Kulturmedium.

## Revendications

1. Gène de la γ-glutamyl transpeptidase ayant la séquence d'ADN décrite dans l'énumération de séquence annexée SEQ ID : N° 1.

2. Molécule d'ADN recombinante comprenant un ADN vecteur et un fragment d'ADN contenant le gène de la γ-glutamyl transpeptidase ayant la séquence d'ADN décrite dans l'énumération de séquence annexée SEQ ID : N° 1.

3. Micro-organisme portant la molécule d'ADN recombinante comprenant un ADN vecteur et un fragment d'ADN contenant le gène de la γ-glutamyl transpeptidase ayant la séquence d'ADN décrite dans l'énumération de séquence annexée SEQ ID : N° 1.

4. Micro-organisme selon la revendication 3, qui est un micro-organisme du genre Bacillus.

5. Micro-organisme selon la revendication 4, qui est le Bacillus subtilis SJ139 (FERM BP-2695).

6. Procédé pour produire la γ-glutamyl transpeptidase qui consiste
(a) à cultiver un micro-organisme du genre Bacillus portant la molécule d'ADN recombinante comprenant un ADN vecteur et un fragment d'ADN contenant le gène de la γ-glutamyl transpeptidase ayant la séquence d'ADN décrite dans l'énumération de séquence annexée SEQ ID : N° 1, dans un milieu de culture approprié;
(b) à accumuler la γ-glutamyl transpeptidase dans le milieu de culture; et
(c) à récupérer ladite γ-glutamyl transpeptidase dudit milieu de culture.
